(19)

**Europäisches Patentamt**

**European Patent Office**

**Office européen des brevets**

(11) **EP 1 583 019 A2**

(12) **EUROPEAN PATENT APPLICATION**

(43) Date of publication:
05.10.2005 Bulletin 2005/40

(51) Int Cl.$^7$: **G06F 19/00**

(21) Application number: **05250531.0**

(22) Date of filing: **01.02.2005**

(84) Designated Contracting States:
**AT BE BG CH CY CZ DE DK EE ES FI FR GB GR
HU IE IS IT LI LT LU MC NL PL PT RO SE SI SK TR**
Designated Extension States:
**AL BA HR LV MK YU**

(30) Priority: **04.02.2004 KR 2004007232**

(71) Applicant: **SAMSUNG ELECTRONICS CO., LTD.
Gyeonggi-do (KR)**

(72) Inventors:
 • **Yeo, Hyung-sok
  Giheung-eub, Yongin-si Gyeonggi-do (KR)**
 • **Kim, Kyung-ho
  Giheung-eub, Yongin-si Gyeonggi-do (KR)**
 • **Whang, Jin-sang
  Jangan-gu, Suwon-si Gyeonggi-do (KR)**
 • **Lee, Jeong-hwan
  Yeongtong-gu, Suwon-si Gyeonggi-do (KR)**

(74) Representative: **Ertl, Nicholas Justin
  Elkington and Fife LLP,
  Prospect House,
  8 Pembroke Road
  Sevenoaks,
  Kent TN13 1XR (GB)**

(54) **System and method for managing growth and development of child**

(57)    There are provided a system for and a method of managing growth and development of a child. The system includes: a biological information measuring module for acquiring at least two biological signals to be used for analyzing growth and development of a user, and identifying the user by analyzing at least one biological signal of the acquired biological signals; and a biological information processing module for evaluating a development state and a growth level of the user in response to the biological signals, storing and managing personal data and the evaluated result of the user.

FIG. 1

**Description**

BACKGROUND OF THE INVENTION

The present invention relates to an apparatus and a method for measuring biological information, and more particularly, to an apparatus and a method for measuring and managing biological information associated with growth and development of a child.

In general, biological information reflecting growth degrees of children include, for example, heights, weights, and so on, and biological information reflecting development states of children include, for example, data on fatness, etc. obtained by measuring body fats of children. The data such as heights, weights, and so on, which are used as barometers for evaluating a growth degree, are the most basic biological information reflecting the growth degrees of human bodies, and the body fat data, which is used as a barometer for evaluating a development state, is widely used as a parameter reflecting nutritive conditions of the human bodies. For example, the body fat data is also important as a parameter for managing fatness or beauty, and can be also used for diagnosing the developments and the nutritive conditions of children or the nutritive conditions of the old or weak.

Generally, the biological information could be obtained by measuring growth data and development data with individual measuring modules. However, conventional biological information measuring apparatuses could supply the biological information such as weights, heights, body fats, etc. only individually, but not comprehensively. That is, the conventional biological information measuring apparatuses did not supply the health data for managing growth and development states of children for each period (for example, daily, weekly, monthly, etc.) and providing solutions such as customized exercise data, diet, rest, etc. suitable for the development states of the children.

Further, since most biological information measuring apparatuses work manually, there are disadvantages that a certain degree of errors always exist. Furthermore, since the measuring apparatuses occupy large spaces, there is a disadvantage that moving and keeping of the measuring apparatuses are not easy.

SUMMARY OF THE INVENTION

According to an aspect of the present invention, there is provided a system for managing growth and development of a child, the system comprising: a biological information measuring module for acquiring at least two biological signals to be used for analyzing growth and development of a user, and identifying the user by analyzing at least one biological signal of the acquired biological signals; and a biological information processing module for evaluating a development state and a growth level of the user in response to the biological signals, storing and managing personal data and the evaluated result of the user.

According to another aspect of the present invention, there is provided a method of managing growth and development of a child, the method comprising: (a) determining whether a user is positioned at a right position for measurement of biological information; (b) identifying the user on the basis of at least one biological signal acquired from an integrated identification-weight-body fat measuring module; (c) loading previously registered data of the user and measuring the biological information from the user; (d) transmitting wirelessly the measured biological information to a biological information processing module; and (e) allowing the biological information processing module to evaluate a development state and a growth level of the user in response to the biological information and store the evaluated result of the user.

The present invention thus provides a system and a method for managing growth and development of a child, the system and the method comprehensively supplying data on a growth and development state of the child, and solutions such as exercise, rest, diet, etc. suitable for a current situation of the child by collecting and analyzing biological information such as height, weight, body fat, etc. of the child and showing time-sequential transition results in the same kind of biological information of the child, the data having been stored for a predetermined period.

The present invention also provides a system and a method for managing growth and development of a child, the system and method being easily used at any place since a space for a biological signal measuring device is minimized to facilitate carrying, moving, and keeping of the biological signal measuring device.

The present invention also provides a computer readable recording medium on which a program for executing the method with a computer is recorded.

BRIEF DESCRIPTION OF THE DRAWINGS

The above and other features and advantages of the present invention will become more apparent by describing in detail exemplary embodiments thereof with reference to the attached drawings in which:

FIG. 1 is a diagram illustrating an outer appearance of a system for managing growth and development of a child

according to an exemplary embodiment of the present invention;

FIG. 2 is a block diagram schematically illustrating a configuration of the system shown in FIG. 1;

FIG. 3 is a block diagram illustrating in detail a height measuring module shown in FIGS. 1 and 2;

FIG. 4 is a diagram illustrating an outer appearance of an identification-weight-body fat measuring module shown in FIGS. 1 and 2;

FIG. 5 is a block diagram illustrating in detail a configuration of the identification-weight-body fat measuring module shown in FIGS. 1 and 2;

FIG. 6 is a block diagram illustrating a configuration of biological information processing module according to an embodiment of the present invention;

FIG. 7 is a block diagram illustrating a whole configuration of a system for managing growth and development of a child to which the biological information processing module shown in FIG. 6 is applied;

FIG. 8 is a block diagram illustrating a configuration of a biological information processing module according to another embodiment of the present invention;

FIG. 9 is a block diagram illustrating a configuration of a fatness and nutritive condition managing system and health information managing system to which the biological information processing module shown in FIG. 8 is applied;

FIG. 10 is a diagram illustrating data flow between the biological information processing module and a data center shown in FIG. 9;

FIG. 11 is a flowchart illustrating a method of measuring and analyzing growth and development of a child according to the exemplary embodiment of the present invention;

FIG. 12 is a flowchart illustrating in detail an operation of determining a right position of a user shown in FIG. 11;

FIG. 13 is a flowchart illustrating in detail an operation of identifying a user shown in FIG. 11;

FIG. 14 is a flowchart illustrating in detail an operation of measuring biological information shown in FIG. 11;

FIG. 15 is a flowchart illustrating in detail an operation of processing and analyzing data shown in FIG. 11;

FIG. 16 is a graph illustrating an operation of determining a positive growth term shown in FIG. 15; and

FIG. 17 is a diagram illustrating screens for analyzing growth and development of a child, according to an exemplary embodiment of the present invention.

## DETAILED DESCRIPTION OF THE INVENTION

Hereinafter, exemplary embodiments of the present invention will be described in detail with reference to the attached drawings.

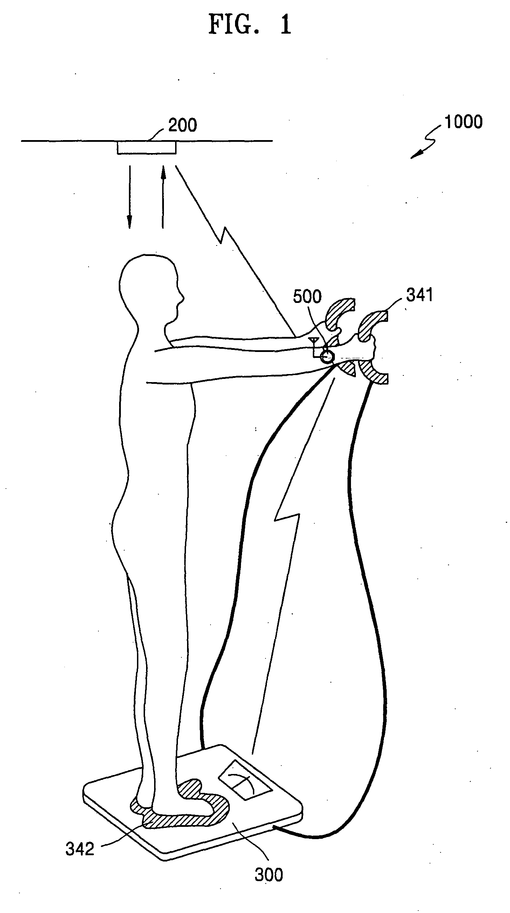

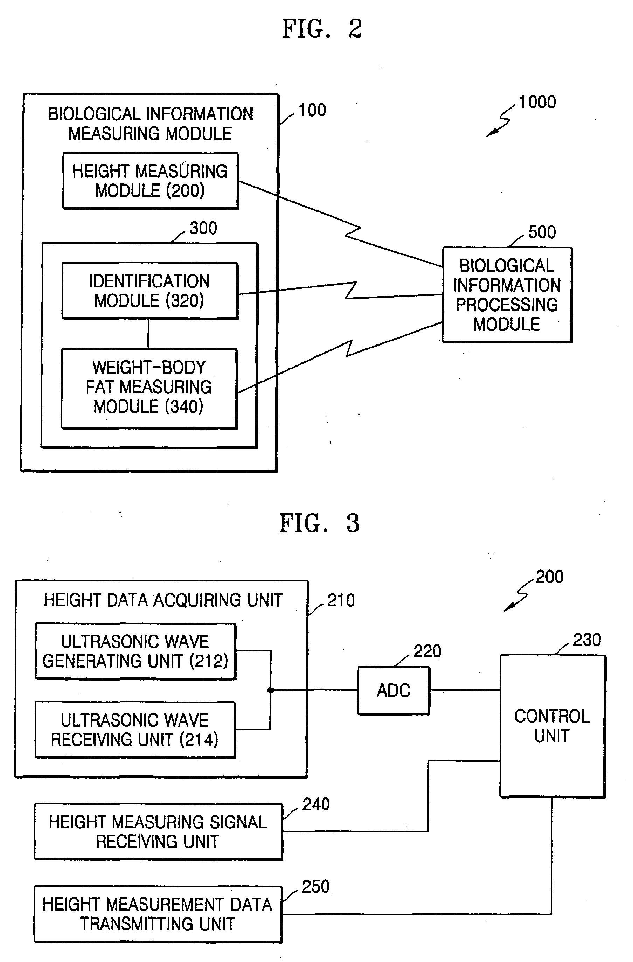

FIG. 1 is a diagram illustrating an outer appearance of a system 1000 for managing growth and development of a child according to an exemplary embodiment of the present invention, and FIG. 2 is a block diagram schematically illustrating a configuration of the system 1000 shown in FIG. 1.

Referring to FIGS. 1 and 2, the system 1000 for managing growth and development of a child roughly comprises a biological information measuring module 100 and a biological information processing module 500, where the biological information measuring module 100 comprises a height measuring module 200 and an identification-weight-body fat measuring module 300. The identification-weight-body fat measuring module 300 comprises an identification module 320 and a weight-body fat measuring module 340. Since each of these modules has a radio communication port, they can communicate with each other wirelessly in a separated situation.

The height measuring module 200 involved in the biological information measuring module 100 measures a height of a user using ultrasonic waves, and transmits wirelessly the measured results by user to the biological information processing module 500. The identification-weight-body fat measuring module 300 analyzes electrocardiogram ECG signals acquired from hand electrodes 341 contacting with palm and foot electrodes 342 contacting with soles of both feet while or before measuring the height with the height measuring module 200, and identifies the user. Then, weight and body fat of the identified user are comprehensively measured, and the measured results for each user are transmitted wirelessly to the biological information processing module 500. In this case, because the identification is automatically recognized through each of the analyzed ECG signals and information on the recognized user is transmitted to the biological information processing module 500, it is not necessary for a user to input data one by one for registering whenever measuring the biological information. Consequently, the biological information processing module 500 can systematically manage growth and development data for each user, even health-relevant information on the corresponding user.

The biological information processing module 500 receives the biological information transmitted from the radio communication port in the biological information measuring module 100, analyzes the biological information, that is, height, weight, body fat data, evaluates a development state and a growth level of the user, and analyzes growth and development variations for each predetermined period. Then, the received biological information and the analyzed results are stored and managed in a predetermined storage area. The biological information processing module 500 is a stand-alone type device, and can be embodied in a PC (Personal Computer), a mobile phone, a wrist watch, a PDA (Personal Digital Assistant), etc.

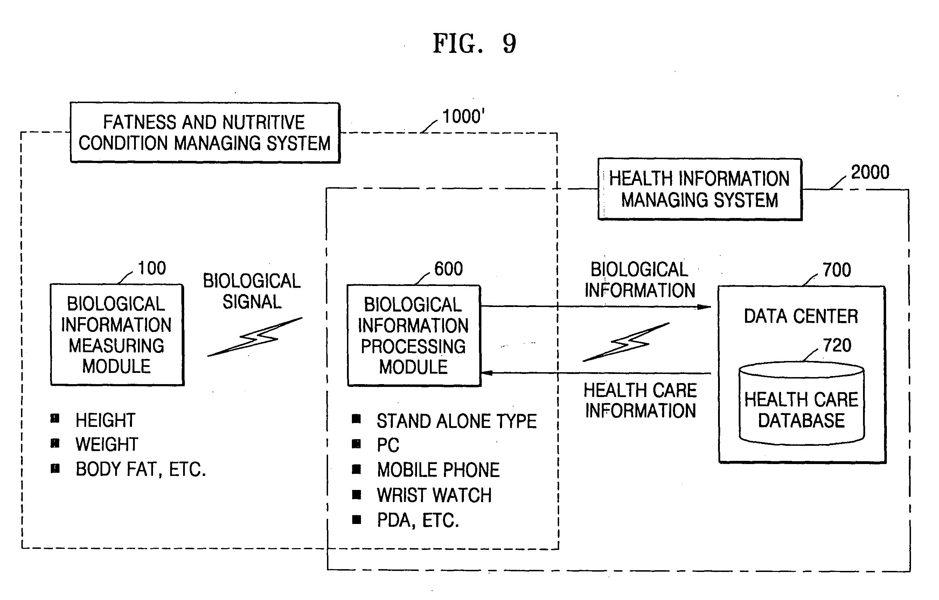

The system 1000 for managing growth and development of a child is portable and detachable at any place such as home or traveling place. Particularly, since the system 1000 performs a function of measuring and managing data of children, it is constructed such that a basic measuring procedure is easy and simple and various biological information can be processed quickly and exactly through a batch procedure. Further, the system 1000 manages the health-relevant data such as fatness and nutritive condition, etc. for each user and each period, as well as managing growth and development of children. Accordingly the system can comprehensively supply additional information such as health information, exercise information, menu, etc. for each period to each user (see FIG. 9). The system can be applied to adults as well.

FIG. 3 is a detailed block diagram of the height measuring module shown in FIGS. 1 and 2. The height measuring module 200 using an ultrasonic wave sensor is portable, and can be easily established. For example, the height measuring module 200 can be placed on the ceiling to measure a user's height in anywhere of a room.

Referring to FIG. 3, the height measuring module 200 comprises a height data acquiring unit 210, an analog-to-digital converter (ADC) 220, a control unit 230, a height measured signal receiving unit 240, and a height measurement data transmitting unit 250.

The height data acquiring unit 210 irradiates ultrasonic waves on a user's head portion using a Doppler's effect and then senses the reflected ultrasonic waves. The height data acquiring unit 210 comprises an ultrasonic wave generating unit 212 for irradiating ultrasonic waves to a user and an ultrasonic wave receiving unit 214 for receiving the ultrasonic waves reflected from the user.

The received analog-type ultrasonic waves acquired from the height data acquiring unit 210 are converted to a digital ultrasonic wave data by the ADC 220. The digital ultrasonic wave data is input to the control unit 230. The control unit 230 comprises one of a microprocessor, a CPU (Central Processing Unit), a DSP (Digital Signal Processor), and another corresponding processor. The control unit 230 responds to height measuring signals received from the height measured signal receiving unit 240 and the ultrasonic wave data, and calculates a user height. At this time, the calculated user height is obtained by subtracting the distance from the ceiling to a top of the user's head after a user positions in a predetermined measuring position from the distance from the ceiling to the upper surface of the identification-weight-body fat measuring module 300 situated in a vertical downward direction of the ceiling. After the user height is calculated in the control unit 230, it is transmitted wirelessly to the biological information processing module 500 through the height measurement data transmitting unit 250.

As described above, since the height measuring module 200 measures a user height to non-contact mode using ultrasonic waves, an error and disadvantage can be reduced, compared with manually measured case. Also, the occupying space of the height measuring device can be also reduced.

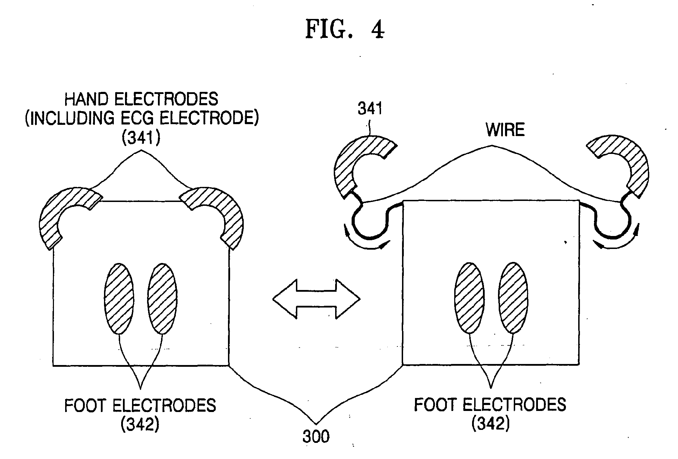

FIG. 4 is a diagram illustrating an outer appearance of the identification-weight-body fat measuring module shown in FIGS. 1 and 2, and FIG. 5 is a detailed block diagram illustrating a configuration of the identification-weight-body fat measuring module shown in FIGS. 1 and 2.

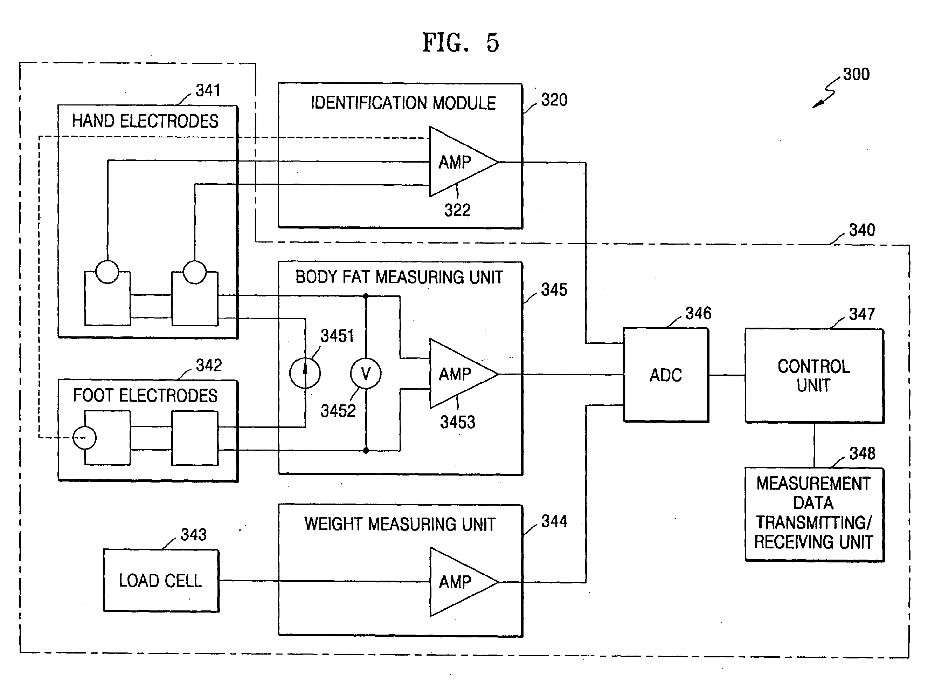

Referring to FIGS. 4 and 5, the identification-weight-body fat measuring module 300 measures all of a user's weight, body fat, and ECG signals at once. A load cell 343 for measuring a weight is included in a main body of the identification-weight-body fat measuring module 300. Further, two foot electrodes 342 for measuring the body fat are at a top portion of the main body of the identification-weight-body fat measuring module 300, and two hand electrodes 341 for measuring the body fat are at two upper corners of the main body of the identification-weight-body fat measuring module 300.

The foot electrodes 342 are fixed to the top portion of the main body of the identification-weight-body fat measuring module 300, but the hand electrodes 341 are wired to the main body of the identification-weight-body fat measuring module 300 to provide flexibility to move. For instance, when a user pulls the hand electrodes 341, the hand electrodes 341 extend through a wire from the main body to a user side. Further, when a user loosens the hand electrodes 341, the wire is rewound into the main body of the identification-weight-body fat measuring module 300 in order for the hand electrodes 341 to return again to the corner portion of the main body.

The hand electrodes 341 and the foot electrodes 342 are commonly used for measuring the body fat and ECG. When a user steps on the foot electrodes 342 and pulls the hand electrodes 341, the identification-weight-body fat measuring module 300 identified the user through measuring the ECG signal before measuring the biological signals, and measures weight of the identified user and bioelectric impedance input from the hand electrodes 341 and the foot electrodes 342. Then, the measured biological signals are transmitted wirelessly to the biological information processing module 500.

That is, the identification-weight-body fat measuring module 300 performs substantially simultaneously operations of identifying a user through measuring the ECG signal, reading a user weight, and measuring the bioelectric impedance using electrodes contacted with the user's hands and feet while measuring a user's height by the height measuring module 200.

The weight-body fat measuring module 340 comprises the hand electrodes 341, the foot electrodes 342, the load cell 343, a weight measuring unit 344, a body fat measuring unit 345, an ADC 346, a control unit 347, and a measurement data transmitting/receiving unit 348. An identification module 320 comprises an ECG measuring unit 322, and shares the hand and foot electrodes 341, 342 as ECG electrodes and processes the ECG signal acquired from the ECG measuring unit 322 by sharing the ADC 346, the control unit 347, and the measurement data transmitting/receiving unit 348.

Detailed operation performed in each module is as follows.

First, the identification module 320 transmits an amplified ECG signal to the ADC 346 after amplifying by a predetermined magnitude the ECG signal input from the hand electrodes 341 and the foot electrodes 342 through the ECG measuring unit 322. An ECG record method used for measuring the ECG signal may be performed through electrodes of a right hand and a left hand by a first standard limb lead, may be performed through electrodes of a right hand and a left foot by a second standard limb lead, and may be performed through electrodes of a left hand and a left foot by a third standard limb lead.

After receiving the digital-type ECG signals from the ADC 346, the control unit 347 analyzes the ECG signal, and identifies a user by comparing the analyzed ECG signal with ECG templates of registered user's. When the input ECG signal is equal with one of the registered ECG signals, the control unit 347 retrieves the identified user's personal data, for example, a user's name, birth date, sex, blood type, basic medical history, and so on, stored at the biological information processing module 500 through the measurement data transmitting/receiving unit 348. As a result, the biological information such as fat condition information, nutritive condition information can be measured without repetitively inputting information required for managing a child's growth and development, for example, data of a user's name, birth date, sex, blood type, basic medical history, and so on. In this case, as information for identifying a user, combined information of weight, bioelectric impedance, body fat, etc. as well as the ECG signal acquired from the user may be also used.

Next, each functional block constituting the weight-body fat measuring module 340 will be operated as follows.

The weight measuring unit 344 receives a user's weight data sensed by the load cell 343 when a user steps on the identification-weight-body fat measuring module 300, amplifies the received data, and transmits to the ADC 346. At this time, data used for measuring weight is data acquired during a few seconds before user's hands contact the hand electrodes 341 after a user steps on the identification-weight-body fat measuring module 300, and the control unit 347 receives digital-type weight data from the ADC 346, calculates average of the received weight data, and outputs the average as the user's weight. Further, the output weight data are transmitted wirelessly to the biological information processing module 500 through the measurement data transmitting/receiving unit 348.

When a user comes in contact with both of the foot electrodes 342 and the hand electrodes 341 of the identification-weight-body fat measuring module 300, the body fat measuring unit 345 measures the bioelectric impedance of the user. A bioelectric impedance measuring method is a method of estimating a water amount, a muscle amount, a fat amount, etc. of a human body by measuring electric resistance or impedance of the human body when the human body contacts with the electrodes 341 and 342 and a weak alternating current flows to the human body. The body fat measuring unit 345 comprises a current source 3451 for allowing predetermined current to flow between two electrode pair 341 and 342, a voltage meter 3452 for measuring voltage between two electrode pair 341 and 342, and an amplifier 3453 for amplifying voltage measured in the voltage meter 3452. Bioelectric impedance data measured in the body fat measuring unit 345 is amplified by the amplifier 3453 and is transmitted to the ADC 346. A control unit 347 receives digital-type bioelectric impedance data from the ADC 346 and analyzes body fat ingredients from the received bioelectric impedance data. The analyzed body fat data is transmitted wirelessly to the biological information processing module 500 through the measurement data transmitting/receiving unit 348.

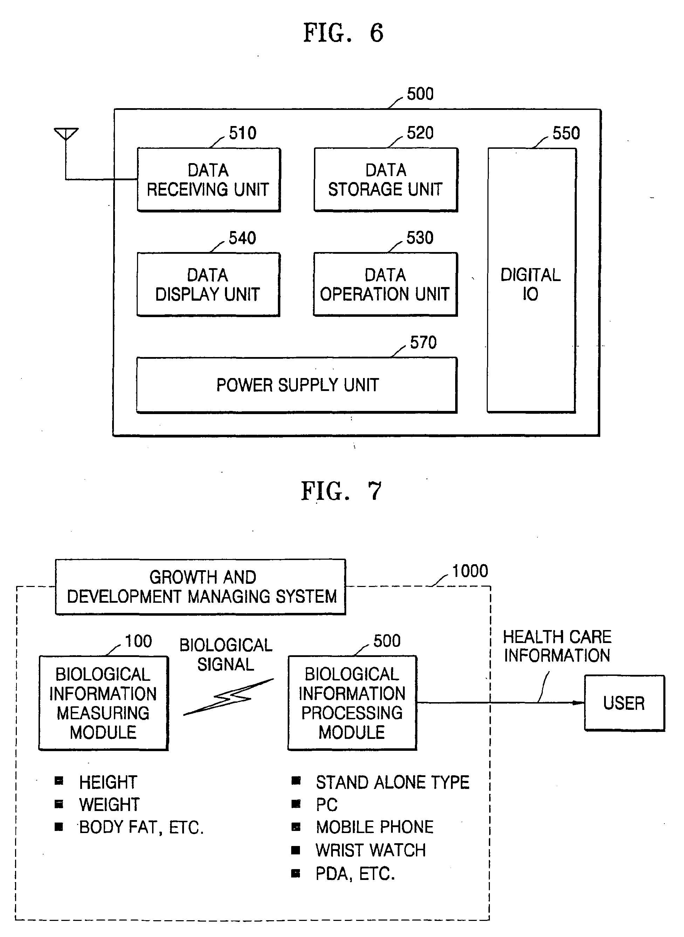

FIG. 6 is a block diagram illustrating a configuration of a biological information processing module according to an embodiment of the present invention, and FIG. 7 is a block diagram illustrating a whole configuration of the system to which the biological information processing module shown in FIG. 6 is applied.

Referring to FIG. 6, the biological information processing module 500 comprises a data receiving unit 510, a data storage unit 520, a data operation unit 530, a data display unit 540, a digital input and output (digital IO) unit 550, and a power supply unit 570. The biological information processing module 500 analyzes, stores, and manages the biological information transmitted wirelessly from the biological information measuring module 100.

The data receiving unit 510 has a radio data receiver and receives the biological information transmitted wirelessly from the biological information measuring module 100. The data storage unit 520 sorts and stores the biological information received from the data receiving unit 510 for each user. The data operation unit 530 analyzes growth and development data of a user (for example, child) through a statistical process and a predetermined analyzing algorithm of the received biological information. The analyzed results by the data operation unit 530 are transmitted to the data storage unit 520 and are stored for each user, and the analyzed results or the biological information stored in the data storage unit 520 are supplied to a user through the data display unit 540. The data display unit 540 has a speaker (not shown) outputting relevant data in a voice and a display device, for instance, liquid crystal display LCD, etc. outputting the analyzed biological information images. The digital IO unit 550 controls input/output of the data receiving unit 510, the data storage unit 520, the data operation unit 530, and the data display unit 540. The power supply unit 570 supplies electric power required for the biological information processing module 500.

Referring to FIG. 7, the biological information processing module 500 together with the biological information measuring module 100 is involved in the system 1000. The biological information processing module 500 receives wirelessly, analyzes, and manages for each user the biological information of height, weight, body fat, etc. measured from the biological information measuring module 100. The biological information processing module 500 can be embodied in an exclusive platform for managing the user's growth and development information and can also be embodied in a mobile phone, a PDA, or a wrist-watch typed potable terminal, etc.

As the growth and development managing system 1000 extends the configuration so that the biological information processing module 500 can interact with a data center (see 700 of FIG. 9) in a remote place as well as the biological information measuring module 100, the system can supply fatness and nutritive condition managing services and health information managing services of adult as well.

The configuration of the biological information processing module having such extended functions will be as follows.

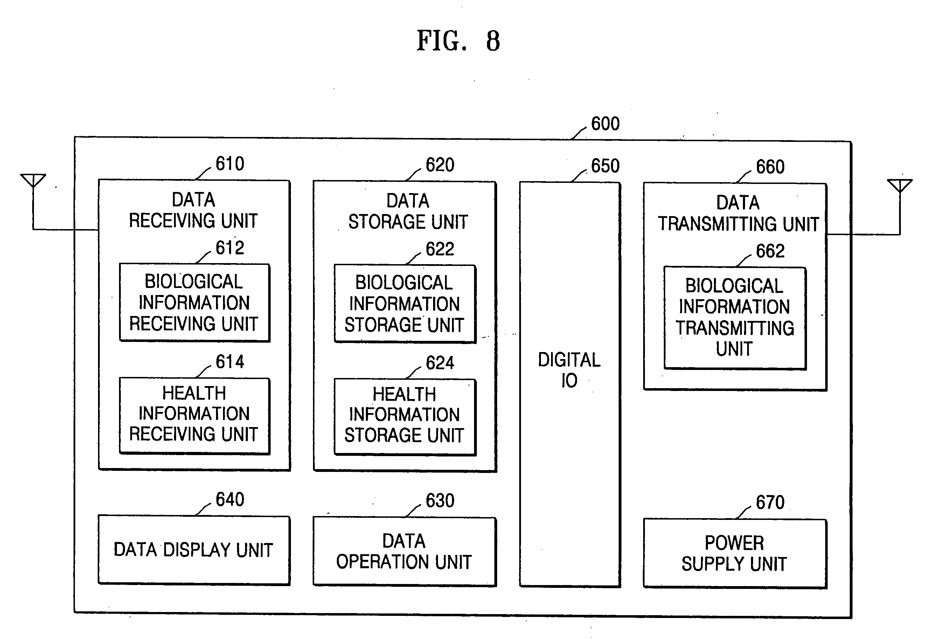

FIG. 8 is a block diagram illustrating a configuration of the biological information processing module according to another embodiment of the present invention, and FIG. 9 is a block diagram illustrating a configuration of a fatness and nutritive condition managing system and a health information managing system to which the biological information processing module shown in FIG. 8 is applied.

Referring to FIG. 8, the biological information processing module 600 performs functions of analyzing the biological information transmitted wirelessly from the biological information measuring module 100 to manage fatness and nutritive conditions, and storing and managing health care information for each user transmitted wirelessly from the data center (see 700 of FIG. 9). The biological information processing module 600 comprises a data receiving unit 610, a data storage unit 620, a data operation unit 630, a data display unit 640, a digital IO unit 650, a data transmitting unit 660, and a power supply unit 670.

The data receiving unit 610 comprises a biological information receiving unit 612 and a health information receiving unit 614. The data receiving unit 610 receives each of the biological information transmitted from the biological information measuring module 100 and the health care information for each user transmitted from the data center. The data storage unit 620 comprises the biological information storage unit 622 and the health information storage unit 624. The data storage unit 620 stores the biological information and the health care information for each user received from the biological information receiving unit 612 and the health information receiving unit 614. , Particularly, since a user identification function is provided to the fatness and nutritive condition managing system 1000' to which the biological information processing module 600 is applied and a health information managing system 2000, the data storage unit 620 manages the biological information and the health care information for each user according to a user recognition result.

The data operation unit 630 analyzes a fatness and nutritive condition management information of a user, for example, a child through statistical process and a predetermined analysis algorithm on the received biological information. The analyzed results are transmitted to the data storage unit 620, are stored for each user, and are transmitted wirelessly to the data center through the digital IO unit 650 and the data transmitting unit 660. On the other hand, the analyzed results or the biological information stored to the data storage unit 620 are supplied to a user through the data display unit 640. The data display unit 640 has a speaker (not shown) to output related information in a voice and a display device, for instance, LCD, etc., to output the analyzed biological information in images.

The data transmitting unit 660 involves a biological information transmission unit 662 and transmits the analyzed results of the biological information to the data center 700. The digital IO unit 650 controls the input/output of the data receiving unit 610, the data storage unit 620, the data operation unit 630, the data display unit 640, and the data transmitting unit 660. The power supply unit 670 supplies electric power required for the biological information processing module 600.

In this way, the biological information processing module 600 shown in FIG. 8 performs functions of processing and managing biological information for each user as in the biological information processing module 500 shown in FIG. 6 and additional functions of exchanging and managing health information such as custom-made health information for each predetermined period for each user based on data of height, weight, body fat, etc., with the data center in a remote place. Therefore, the module can systematically supply the growth and development data for each user, and even health-relevant information on corresponding user.

Referring to FIG. 9, the biological information processing module 600 together with the biological information measuring module 100 is involved in the fatness and nutritive condition managing system 1000' and the health information managing system 2000. The biological information processing module 600 receives wirelessly, analyzes, and manages for each user the biological information of height, weight, body fat, etc. measured from the biological information measuring module 100. Further, the biological information processing module 600 together with the data center 700 is involved in the health information managing system 2000. The biological information processing module 600 analyzes, stores, and manages the biological information transmitted wirelessly from the biological information measuring module 100, and stores and manages the health care information for each user transmitted wirelessly from the data center 700. In this way, the biological information processing module 600 can be embodied in an exclusive platform, a mobile phone, a PDA, a wrist-watch typed potable terminal, etc. managing the user growth and development data or fat and nutritive management data and health-relevant information.

The data center 700 is situated in a remote place of the biological information processing module 600, involves a health care data database 720, and packs the health-relevant information for each user to a database.

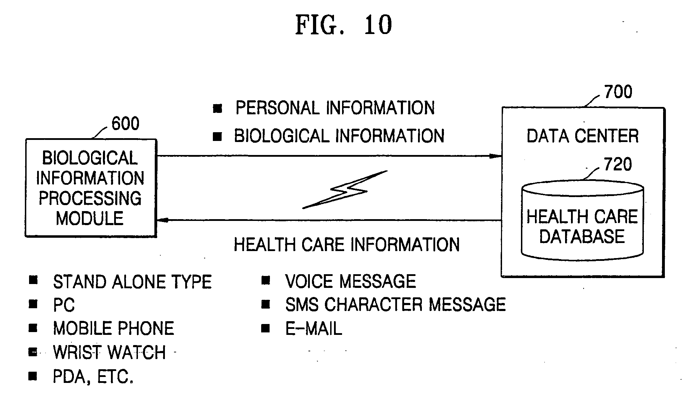

FIG. 10 is a diagram illustrating data flow between the biological information processing module 600 and the data center 700 shown in FIG. 9.

Referring to FIG. 10, the data center 700 analyzes the health care information of a user for each period, for example, daily, weekly, monthly, based on the biological information, for example, height, weight, body fat data, and the user personal information, for example, sex, age, basic medical history, transmitted from the biological information processing module 600, performs statistic processes and analytic algorithms on the health care information and the user personal information, and then separates the performed results for each user and stores the separated results to the health care information database 720. Further, the data center 700 can make the biological information processing module 600 to integratedly supply the health care information to a user by transmitting back the health care information for each user stored in the health care information database 720 to the biological information processing module 600. The health care information can be supplied as a voice message, a short message service SMS, an e-mail, etc., to a user or a user can retrieve relevant data by logging in the data center 700.

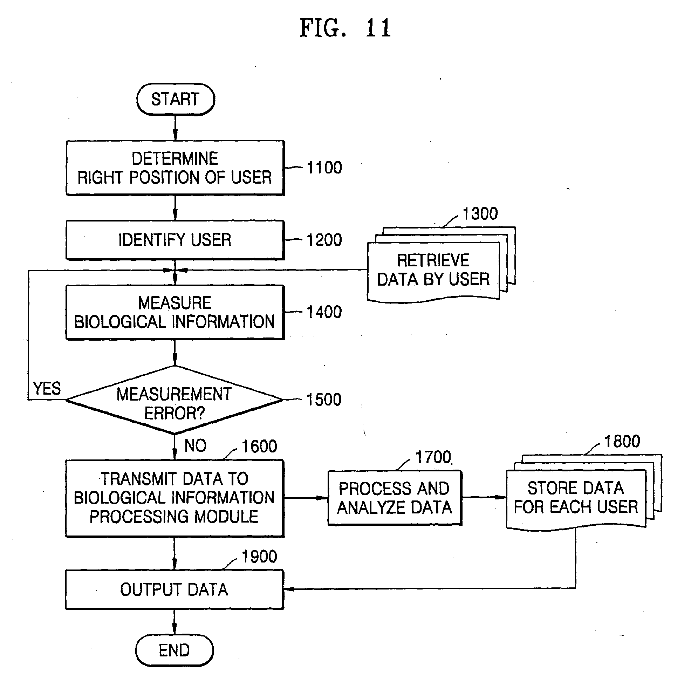

FIG. 11 is a flowchart illustrating a growth and development measuring and analyzing method to be performed in a system 1000 for managing growth and development of a child, according to an exemplary embodiment of the present invention.

Referring to FIG. 11, the system 1000 for managing growth and development of a child determines whether a user stands in a right position for measuring the biological information (operation 1100), and identifies a user by acquiring the ECG signal from the user through the integrated identification-weight-body fat measuring module 300 (operation 1200). At this time, combination of weight information, bioelectric impedance information, and body fat information, etc. acquired from the user as well as the ECG signal may be used in the identifying process. When a user is identified in operation 1200, the registered data related to the user is retrieved (operation 1300), and thus a pre-process for measurement is completed.

Next, the biological information, for example, height, weight, body fat data, etc., is measured through the biological information measuring module 100 (operation 1400), and it is determined whether there is an error in the measured biological information (operation 1500). When there is an error in the measured biological information, the process returns to operation 1400 and the biological information is measured again, but when there is no error in the measured biological information, the measured biological information is transmitted wirelessly to the biological information processing module 500 (operation 1600).

Next, after the biological information processing module 500 processes and analyzes the biological information transmitted from the biological information measuring module 100 (operation 1700), the analyzed results are stored for each user (operation 1800). The analyzed results stored in operation 1800 may be stored for each user in the biological information processing module 500, and may be stored to a database at the data center 700. Such biological information stored for each user is supplied to a user in a voice message, a SMS, an e-mail, etc., through the biological information processing module 500 (operation 1900), and a user can retrieve relevant data by logging in the data center 700.

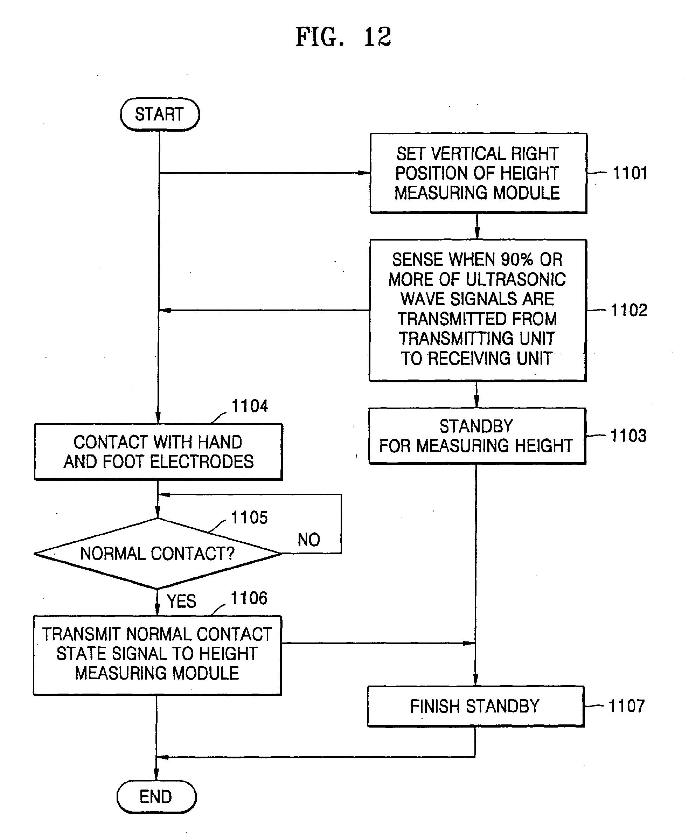

FIG. 12 is a detailed flow chart for determining whether a user stands at a right position shown in FIG. 11.

Referring to FIG. 12, the system 1000 sets a vertical right position of the height measuring module 200 determining whether a user stands at a right position (operation 1101). Vertical right position setting process performed in operation 1101 is a process of normally implementing a the ultrasonic wave generating unit 212 and the ultrasonic wave receiving unit 214 on the ceiling and the floor of a room for measuring height and is performed when initializing the system 1000.

Next, when 90% or more of ultrasonic wave signals generated from the ultrasonic wave generating unit 212 is sensed in the ultrasonic wave receiving unit 214, it is determined as the height measuring module 200 is normally implemented (operation 1102).

When it is determined as the height measuring module 200 is normally implemented in operation 1102, a process of measuring a height is on standby (operation 1103), the hand and the foot electrodes 341 and 342 of the integrated identification-weight-body fat measuring module 300 are contacted to a user's hands and feet, respectively (operation 1104). Then, it is determined whether the contact state of electrodes is normal by confirming whether the hand and the foot electrodes 341 and 342 of the identification-weight-body fat measuring module 300 normally contacted to a user's hands and feet (operation 1105). For example, if 4 or 8 electrodes normally contact to a user's hands and feet when measuring body fat, a voltage drop starts from a saturated bioelectric impedance. The contact state of the electrodes is determined to normal if there is no abnormality after a few seconds pass from the start of the voltage drop.

In operation 1105, when the contact state of electrodes is determined as normal, the integrated identification-weight-body fat measuring module 300 transmits signals of which the contact state of the electrodes is normal to the ultrasonic wave generating unit 212 in the height measuring module 200 (operation 1106), the height measuring module 200 on a standby mode for measurement finishes the standby mode and completes preparation for measurement (operation 1107).

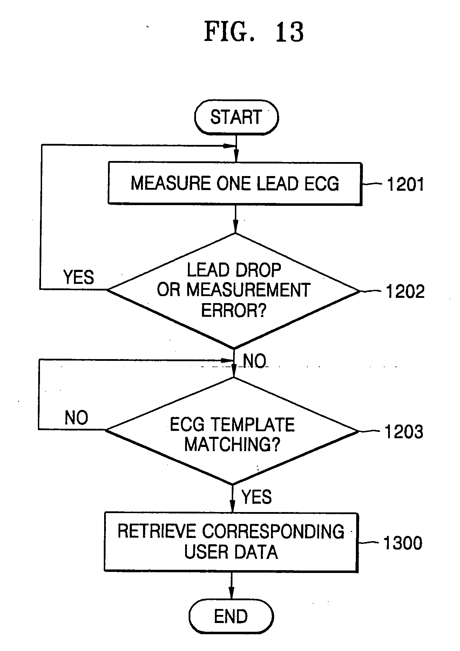

FIG. 13 is a detailed flow chart illustrating a user identifying process shown in FIG. 11.

Referring to FIG. 13, in order to identify a user, the identification-weight-body fat measuring module 300 in the system 1000 measures an ECG signal through the hand electrodes 341 and the foot electrodes 342 contacted to the user's hands and feet (operation 1201). It is also examined whether leads connected to the hand and foot electrodes are dropped or there is an error in measuring the ECG signal (operation 1202). When there is an error in measuring the ECG signal in operation 1202, the process returns to operation 1201 and the ECG signal is measured again. When there is no error in measuring the ECG signal, however, it is determined whether there is a correspondence by comparing the ECG signals measured from the ECG measuring unit 322 with the previously stored ECG templates (operation 1203).

In this case, the ECG templates to be used for identifying a user may be stored to a personal data storage unit in the identification-weight-body fat measuring module 300, or may be stored at the biological information processing module 500 or the data center 700 in a remote place according to the system configuration. In the present invention, the personal data storage unit can interact with the biological information measuring module 100 by implementing the personal data storage unit in the biological information processing module 500. That is, the personal data storage unit may be included in an integrated form to the biological information processing module 500, or may be embodied in a portable electronic record media such as an electronic record card.

Next, when there is the corresponding ECG template in operation 1203, the identification-weight-body fat measuring module 300 retrieves the user data, for example, name, birth date, sex, blood type, basic medical history, etc. from the biological information processing module 500 (operation 1300).

That is, in order to identify a user, the identification-weight-body fat measuring module 300 records ECG data acquired for a predetermined time by the standard limb lead of ECG, and the identifying process is completed by retrieving the most interrelated user health information record through an operation to compare the recorded user ECG data with the previously input ECG templates for each user. A technology to use an ECG signal for identifying a user is disclosed in the article, "One-Lead ECG for Identity Verification" published the 2nd Joint Conference of the IEEE Engineering in Medicine and Biology Society and the Biomedical Engineering Society by T.W. Shen, W.J. Tompkins, and Y.H.Hu in 2002.

The ECG recording for identification is continued for about 20 seconds to 30 seconds when heart beat fluctuates between 20 and 30 times. When the recording is completed, the interrelationship is analyzed by using a training algorism such as a template matching and neural network, etc. QRS onset, P duration, QRS duration, R duration, S duration, ST slope, QRS p-p amplitude, T amplitude, and ST amplitude, etc. are used as ECG matching parameters for the Template matching. A user recognition method through training ECG signals is disclosed in the paper, "ECG analysis: A new approach in human identification", IEEE Proc. Instrumentation & Measurement Technology Conference, to be presented in Venice, Italy by Biel, L., Pettersson, O., Philipson, L., and Wide, P. in 1998. However, the ECG signal is just an example signal adapted in a user recognition method, and weight, bioelectric impedance, and body fat information, and so on acquired from a user as well as the ECG signal may be also used in user recognition.

According to a user recognition method of the present invention, whenever measuring the biological information, a user is automatically identified through the ECG signal, and health-relevant information as well as the growth and development information of each identified user, can be systematically managed without inputting a user's personal data.

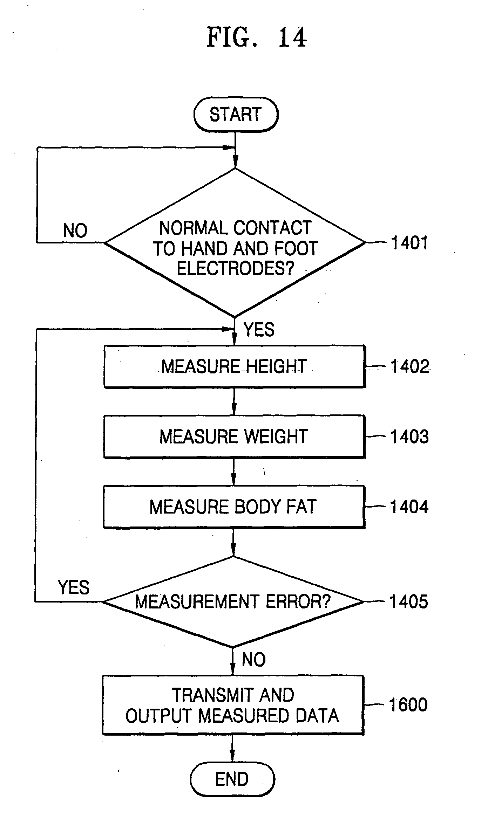

FIG. 14 is a detailed flow chart illustrating the biological information measuring process shown in FIG. 11.

Referring to FIG. 14, in order to measure the biological information, the identification-weight-body fat measuring module 300 in the system 1000 determines whether a contact state of the hand electrodes 341 and the foot electrodes 342 contacted to a user hands and feet is normal (operation 1401). When there is an error in the contact state of the hand and foot electrodes 341 and 342 in operation 1401, a simple voice message requesting to measure again is output. However, when the contact state of the hand and foot electrodes 341 and 342 is normal, the user height is measured through the height measuring module 200 mounted on the ceiling (operation 1402), and the user's weight is measured through the identification-weight-body fat measuring module 300 (operation 1403).

After a bioelectric impedance is measured from the hand and foot electrodes 341 and 342 contacted to the user hands and feet, a body fat is calculated using the measured bioelectric impedance; height information measured in operation 1402, and weight information measured in operation 1403 (operation 1404).

Then, it is determined whether there was a measurement error (operation 1405). When there was no measurement error, the measured data is transmitted wirelessly to the biological information processing module 500 (operation 1600).

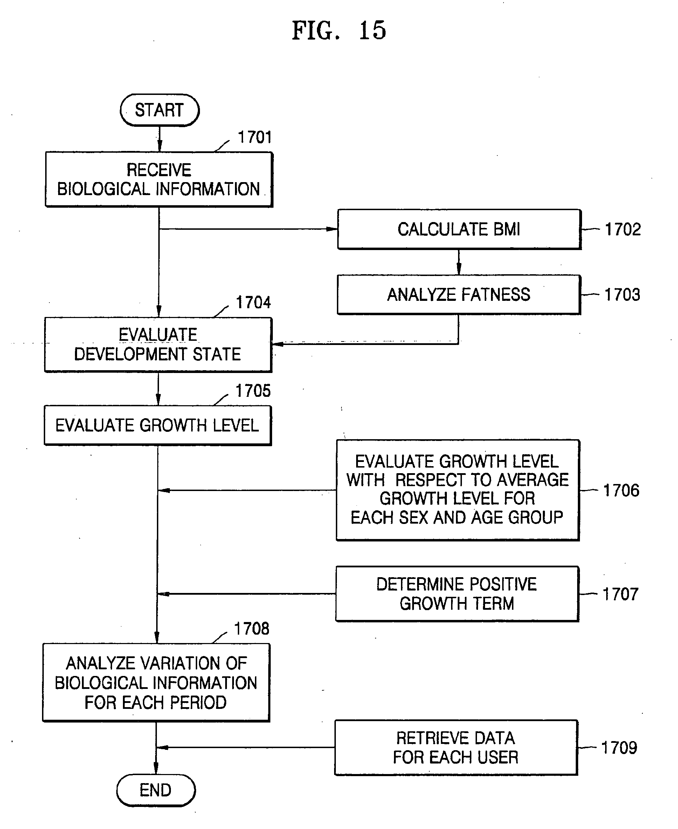

FIG. 15 is a detailed flow chart illustrating the data processing and analyzing process shown in FIG. 11.

Growth and development evaluation performed by the system 1000 is divided into a development state evaluation, a growth level evaluation, a positive growth entry term and positive growth term determination, and an analysis of variation transition in growth and development for each period as shown in Table 1 below.

| Data processing | Method |
|---|---|
| Evaluation of development state | 1) weight analysis and fatness determination by BMI calculation (weight (kg)/ square of height (m)); weight analysis and fatness determination based on BMI table by sex and age of corresponding year |
| | 2) fatness determination by bioelectric impedance measuring method |
| Analysis of growth level evaluation | express the difference based on average height and weight by sex and age as percentage |
| | * growth level vs average height by sex and age = {(average height by sex and age-user's height)/(average height by sex and age)}*100 |
| | * growth level vs average weight by sex and age = {(average weight by sex and age-user's weight)/ (average weight by sex and age)}*100 |
| Determination of positive growth entry term and positive growth term | * the inflection point portion where the slope of a height increment rate curve in recent 6 months since 6 age is converted to positive (+) from negative (-) or zero (0) is determined as a positive growth entry term, and based on this, when the slope of an increment rate curve maintains the positive slope for six months or more or is expanded to more sharp positive slope, it is determined as a positive growth term. |
| Analysis of variation in biological information and development state for each period | Variation is output as a graph over a predetermined period by cumulatively summing weight, height, body fat percentage, and fatness data for each period. |

Referring to FIG. 15, in order to evaluate the growth and development of children as shown in Table 1, the biological information processing module 500 in the system 1000 receives the biological information, that is, weight, body fat, height, etc., measured from the identification-weight-body fat measuring module 300 (operation 1701), and calculates BMI (Body Mass Index) as in the following Equation 1 (operation 1702).

(Equation 1)

$$BMI = weight\ (kg)/\ square\ of\ height\ (m)$$

After BMI is calculated in operation 1702, weight and fatness are analyzed based on a BMI table for sex and age (operation 1703). The BMI table used at this time can replace with the BMI table updated in the nearest time to corresponding year.

Table 2

| below shows an example of a BMI table. | | | | |
|---|---|---|---|---|
| Age | Overweight | | Fatness | |
| 18 or more | 25 to 29 | | 30>BMI | |
| 4 | male | 17.56 | Male | 19.29 |
| | female | 17.28 | Female | 19.15 |
| 6 | male | 17.56 | Male | 19.78 |
| | female | 17.34 | Female | 19.65 |
| 8 | male | 18.44 | Male | 21.60 |
| | female | 18.35 | Female | 21.57 |
| 10 | male | 19.84 | Male | 24.00 |
| | female | 19.86 | Female | 24.11 |
| 12 | male | 21.22 | Male | 26.02 |
| | female | 21.68 | Female | 26.67 |
| 14 | male | 22.62 | Male | 27.63 |
| | female | 23.34 | Female | 28.57 |
| 16 | male | 23.90 | Male | 28.88 |
| | female | 24.37 | Female | 29.43 |
| 18 | male | 25.00 | Male | 30.00 |
| | female | 25.00 | Female | 30.00 |

The analysis of fatness of children performed in operation 1703 is largely divided into determination of fatness based on the BMI table and determination of fatness based on the bioelectric impedance. That is, in operation 1703, after determination of fatness based on BMI table is firstly performed, determination of fatness based on the bioelectric impedance is selectively performed, so that more correct fatness information of a user can be obtained.

A bioelectric impedance measuring method is to flow minute current of which a user cannot sense to the user's hands and feet after attaching 4 electrodes typically on ankle, foot, wrist, and hand and to detect voltage from wrist and ankle. Then, the method is to calculate a body fat percentage from electric conductivity of a human organ, namely, the measured voltage.

It is known that the bioelectric impedance measuring method has high interrelation (r = 0.90 ~ 0.94) with the body fat percentage obtained through an underwater weight measuring method. However, because of a feature of a body fat percentage equation, the body fat percentage of a thin person tends to be overestimated. Therefore, in order to solve such problem, the present invention determines fatness once more based on bioelectric impedance of a user having overweight and fatness after estimating overweight and fatness through the BMI, so that reliability of health information on the user development state is enhanced.

Next, after the fatness is analyzed in operation 1703, the user development state is evaluated based on the analyzed result (operation 1704). In operation 1704, subjects are divided in three groups having low, normal, and high body fat percentage, to determine the development state of users belonging to each group.

Table 3 below shows definitions of three groups based on the body fat percentage.

| Body fat percentage | Group to be evaluated |
|---|---|
| less than 10% | low |
| 10% to 14% | normal |
| 15% or more | high |

Next, evaluation on user's growth level is performed (operation 1705), and the user's growth level vs average growth by sex and age is evaluated (operation 1706). The evaluation of the growth level performed in operation 1706 is divided into evaluation of the growth level vs average height by sex and age and evaluation of the growth level vs average weight by sex and age, and both evaluations are calculated by the following equations, respectively.

(Equation 2)

Growth level vs average height by sex and age = {(average height by sex and age - user's height) / (average height by sex and age)} * 100 (%)

(Equation 3)

Growth level vs average weight by sex and age = {(average weight by sex and age - user's weight) / (average height by sex and age)} * 100 (%)

As described in Equations 2 and 3, in operation 1706, average growth information (height, and weight) and a user's growth information are compared with and the compared result is represented by percentage, so that the growth level is evaluated.

Next, it is determined whether the growth level corresponds to a positive growth term (operation 1707).

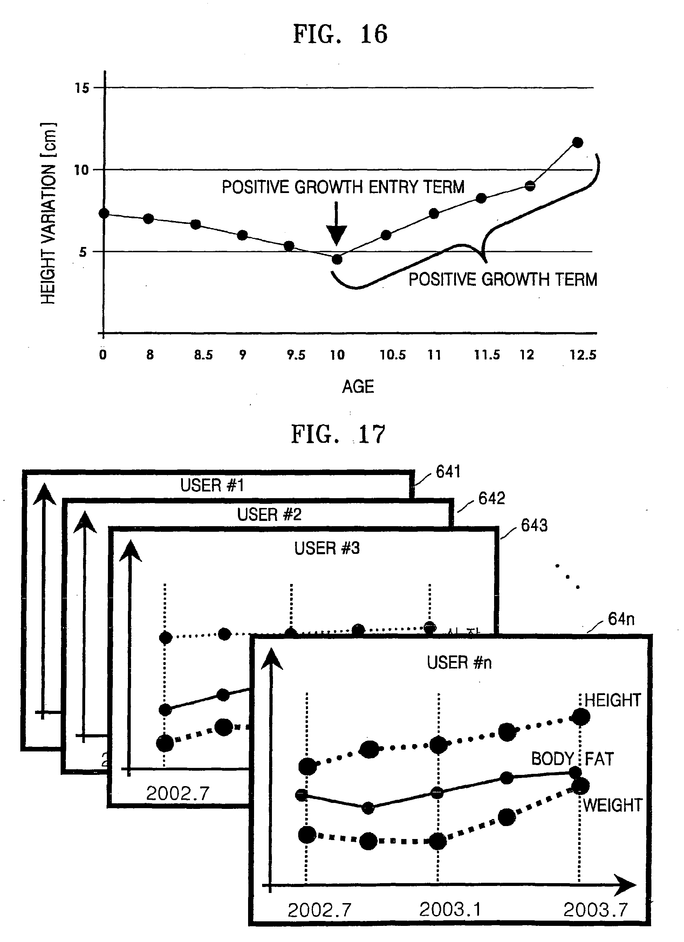

FIG. 16 is a graph illustrating the positive growth term determining process shown in FIG. 15.

Referring to FIG. 16, when the slope of a height increment rate curve in recent 6 months since a user's age becomes 6 is converted to a positive (+) value from a negative (-) value or zero (0), the inflection point portion is determined as a positive growth entry term, and based on this, when the slope of the curve maintains the positive slope for six months or more or is spread to more sharp positive, it is determined as a positive growth term.

Referring again to FIG. 15, after it is determined as the positive growth term in operation 1707, the variation in biological information and development state for period is analyzed (operation 1708), and data for user are retrieved (operation 1709).

That is, in analyzing biological information for user, the system 1000 does not only support to analyze instant data, but also stores the analyzed results on the development state and growth level for user. Then the system 1000 accumulatively sums the analyzed results for each period and each user, and analyzes the variations of the accumulated results.

FIG. 17 is a diagram illustrating a growth and development analysis displays according to an exemplary embodiment of the present invention.

Referring to FIG. 17, the system 1000 provides tendency to the changes of the biological information for each period for each user and the analyzed results thereof. Further, the system provides health information related to the tendency to changes as well as exercise prescription, diet information, etc., required for each user. Furthermore, the system can interact with the data center 700 so as to manage the health information in a remote place, so that it is possible to provide information on sports center for exercise, information on specialized medical clinics related to disease, and a function of making a reservation to see a doctor.

As described above, the system 1000 for managing growth and development of a child collects and analyzes the biological information of height, weight, body fat, etc., for user, and shows time sequential changes of the same kind of biological information which have been stored for a predetermined period for user, so that the system can comprehensively provide information on child's growth and development state and information on a solution such as exercise, rest, diet, etc., suitable for each situation when measuring. Such health information management can be expanded to adults as well as children. Furthermore, the system can minimize a size of device and space required for measuring biological signals, whereby carrying, moving and storing of the system are easy, so that the system can easily be used at a house or a tour place.

The present invention can also be embodied as computer readable codes on a computer readable recording medium. The computer readable recording medium is any data storage device that can store data which can be thereafter read by a computer system. Examples of the computer readable recording medium include read-only memory (ROM), random-access memory (RAM), CD-ROMs, magnetic tapes, floppy disks, optical data storage devices, and carrier waves such as data transmission through the Internet. The computer readable recording medium can also be distributed over network coupled computer systems so that the computer readable code is stored and executed in a distributed fashion.

While the present invention has been particularly shown and described with reference to exemplary embodiments thereof, it will be understood by those skilled in the art that various changes in form and details may be made therein without departing from the scope of the present invention as defined by the appended claims.

**Claims**

1. A system for managing growth and development of a child, the system comprising:

   a biological information measuring module for acquiring at least two biological signals to be used for analyzing growth and development of a user, and identifying the user by analyzing at least one biological signal of the acquired biological signals; and
   a biological information processing module for evaluating a development state and a growth level of the user in response to the biological signals, storing and managing personal data and the evaluated result of the user.

2. The system according to claim 1 or 2, further comprising a data center for receiving the biological information, the personal data, and the evaluated result of the development state and the growth level of the user from the biological information processing module, analyzing information required for a health condition and a health care of the user, and supplying the health-relevant information of the user at every predetermined period.

3. The system according to claim 2, wherein the biological information processing module further comprises;
   a data transmitting unit for transmitting wirelessly the biological information, the personal data, and the evaluated result of the development state and the growth level of the user to the data center; and
   a data receiving unit for receiving the health-relevant data supplied from the data center and providing the health-relevant data to the user.

4. The system according to claim 2 or 3, wherein the data center comprises a health care database for storing the health care data for each user, and
   the health care data is supplied to the biological information processing module in any one among a voice message, a short message service, and an electronic mail.

5. The system according to any preceding claim, wherein the biological information measuring module comprises:

   a height measuring module for measuring height of the user using ultrasonic waves; and
   an integrated identification-weight-body fat for measuring module measuring an electrocardiogram, weight, and a bioelectric impedance of the user, identifying the user by analyzing the measured electrocardiogram, and analyzing a body fat value from the bioelectric impedance.

6. The system according to claim 5, wherein the height measuring module comprises:

   a height data acquiring unit for applying the ultrasonic waves to a head portion of the user and sensing the reflected ultrasonic waves;
   an analog-to-digital converter for converting the sensed ultrasonic waves into digital data;
   a control unit for calculating the height of the user in response to the converted ultrasonic wave data; and
   a data transmitting unit for transmitting wirelessly the calculated height data to the biological information processing module.

7. The system according to claim 5 or 6, wherein the identification-weight-body fat measuring module comprises:

   an identification module for identifying the user by comparing the measured electrocardiogram with the previously-registered electrocardiogram templates; and
   a weight-body fat measuring module for measuring the weight and the bioelectric impedance of the user and analyzing the body fat value from the measured bioelectric impedance.

8. The system according to claim 7, wherein the weight-body fat measuring module comprises:

   hand electrodes for coming in contact with hands of the user and acquiring the electrocardiogram and bioelectric impedance signal;
   foot electrodes for coming in contact with feet of the user and acquiring the electrocardiogram and bioelectric impedance signal;

a load cell for coming in contact with the feet of the user and acquiring the weight signal;

a weight measuring unit for amplifying the weight signal acquired by the load cell;

a body fat measuring unit for measuring the bioelectric impedance signal by allowing a predetermined amount of current to flow in the hand electrodes and the foot electrodes and measuring voltages across the electrodes, and amplifying the measured bioelectric impedance signal;

an analog-to-digital converter for converting the amplified weight signal and the amplified bioelectric impedance signal into digital weight data and digital bioelectric impedance data, respectively;

a control unit for calculating the weight of the user by averaging the digital weight data, and calculating the body fat by analyzing the digital bioelectric impedance data; and

a data transmitting/receiving unit for transmitting wirelessly the weight and the body fat data calculated by the control unit to the biological information processing module and receiving the personal data of the user from the biological information processing module.

**9.** The system according to claim 8, wherein the identification module comprises an electrocardiogram measuring unit for amplifying the electrocardiogram acquired through the hand electrodes and the foot electrodes,

wherein the electrocardiogram amplified by the electrocardiogram measuring unit are compared with the electrocardiogram templates in the control unit in order to identify the user, and

the personal data is received by the data transmitting/receiving unit.

**10.** The system according to any preceding claim, wherein the biological information processing module comprises:

a data receiving unit for receiving wirelessly the biological information transmitted from the biological information measuring module;

a data storage unit for storing the received biological information for each user;

a data operation unit for analyzing the biological information, evaluating the development state and the growth level, and analyzing tendencies of variations in growth and development of the user by period;

a data display unit for outputting the analyzed result to the user;

a digital input and output controller for controlling the data input and output of the data receiving unit, the data storing unit, the data operation unit, and the data display unit; and

a power supply unit for supplying electric power required for the biological information processing module.

**11.** The system according to any preceding claim, wherein the biological information processing module has any one among a stand-alone type device, a personal computer, a mobile phone, a wrist watch, and a personal digital assistant.

**12.** A method of managing growth and development of a child, the method comprising:

determining whether a user is positioned at a right position for measurement of biological information;

identifying the user on the basis of at least one biological signal acquired from an integrated identification-weight-body fat measuring module;

retrieving previously registered data of the identified user and measuring the biological information from the user;

transmitting wirelessly the measured biological information to a biological information processing module; and

allowing the biological information processing module to evaluate a development state and a growth level of the user in response to the biological information and store the evaluated result of the user.

**13.** The method according to claim 12, further comprising:

allowing the biological information processing module to transmit wirelessly the biological information, personal data, and the evaluated result of the development state and growth level of the user to a data center; and

allowing the data center to receive and analyze the biological information, the personal data, and the evaluated result of the development state and growth level of the user, and to supply the analyzed health-relevant information at every predetermined period to the biological information processing module.

**14.** The method according to claim 12 or 13, wherein the evaluating comprises:

calculating a body mass index in response to the biological information and analyzing a weight and a fatness on the basis of a body mass index table for each sex and age;

analyzing the weight and the fatness of the user on the basis of bioelectric impedance measured from the user when the fatness is above a predetermined value;

evaluating the growth level of the user on the basis of average heights and average weights for each sex and age;

determining a positive growth entry term and a positive growth term in consideration of a increment rate in height of the user; and

analyzing variation of the biological information by period by analyzing a tendency of the analyzed results for a predetermined period.

15. The method according to claim 14, wherein after the user is over 6 years old, the positive growth entry term is represented by an inflection point where a slope of a height increment rate curve for latest six months is converted into a positive (+) value from a negative (-) value or zero (0).

16. The method according to claim 14 or 15, wherein the positive growth term is a term when the slope of the curve maintains the positive slope for six months or more or when the slope of the curve has a sharper slope with respect to the positive growth entry term.

17. A computer readable recording medium on which a program for executing a method according to any one of claims 12 to 16 is recorded.

FIG. 1

## FIG. 2

BIOLOGICAL INFORMATION
MEASURING MODULE — 100

HEIGHT MEASURING
MODULE (200)

— 300

IDENTIFICATION
MODULE (320)

WEIGHT-BODY
FAT MEASURING
MODULE (340)

— 1000

— 500

BIOLOGICAL
INFORMATION
PROCESSING
MODULE

## FIG. 3

HEIGHT DATA ACQUIRING UNIT — 210

ULTRASONIC WAVE
GENERATING UNIT (212)

ULTRASONIC WAVE
RECEIVING UNIT (214)

— 200

— 220

ADC

— 230

CONTROL
UNIT

HEIGHT MEASURING SIGNAL
RECEIVING UNIT — 240

HEIGHT MEASUREMENT DATA
TRANSMITTING UNIT — 250

FIG. 4

HAND ELECTRODES
(INCLUDING ECG ELECTRODE)
(341)

341

WIRE

FOOT ELECTRODES
(342)

FOOT ELECTRODES
(342)

300

## FIG. 5

EP 1 583 019 A2

FIG. 6

500

510
DATA
RECEIVING UNIT

520
DATA
STORAGE UNIT

550
DIGITAL
IO

540
DATA
DISPLAY UNIT

530
DATA
OPERATION UNIT

570
POWER SUPPLY UNIT

FIG. 7

GROWTH AND
DEVELOPMENT MANAGING SYSTEM

1000

100
BIOLOGICAL
INFORMATION
MEASURING
MODULE

BIOLOGICAL
SIGNAL

500
BIOLOGICAL
INFORMATION
PROCESSING
MODULE

HEALTH CARE
INFORMATION

USER

- HEIGHT
- WEIGHT
- BODY FAT, ETC.

- STAND ALONE TYPE
- PC
- MOBILE PHONE
- WRIST WATCH
- PDA, ETC.

# FIG. 8

A block diagram labeled 600 containing:

- DATA RECEIVING UNIT (610), containing:
  - BIOLOGICAL INFORMATION RECEIVING UNIT (612)
  - HEALTH INFORMATION RECEIVING UNIT (614)
- DATA STORAGE UNIT (620), containing:
  - BIOLOGICAL INFORMATION STORAGE UNIT (622)
  - HEALTH INFORMATION STORAGE UNIT (624)
- DIGITAL IO (650)
- DATA TRANSMITTING UNIT (660), containing:
  - BIOLOGICAL INFORMATION TRANSMITTING UNIT (662)
- DATA DISPLAY UNIT (640)
- DATA OPERATION UNIT (630)
- POWER SUPPLY UNIT (670)

EP 1 583 019 A2

# FIG. 9

FATNESS AND NUTRITIVE CONDITION MANAGING SYSTEM

1000'

HEALTH INFORMATION MANAGING SYSTEM — 2000

100
BIOLOGICAL INFORMATION MEASURING MODULE

- HEIGHT
- WEIGHT
- BODY FAT, ETC.

BIOLOGICAL SIGNAL

600
BIOLOGICAL INFORMATION PROCESSING MODULE

- STAND ALONE TYPE
- PC
- MOBILE PHONE
- WRIST WATCH
- PDA, ETC.

BIOLOGICAL INFORMATION

HEALTH CARE INFORMATION

700
DATA CENTER

720
HEALTH CARE DATABASE

EP 1 583 019 A2

# FIG. 10

- PERSONAL INFORMATION
- BIOLOGICAL INFORMATION

600

BIOLOGICAL
INFORMATION
PROCESSING
MODULE

700

DATA CENTER

720

HEALTH CARE
DATABASE

HEALTH CARE INFORMATION

- STAND ALONE TYPE
- PC
- MOBILE PHONE
- WRIST WATCH
- PDA, ETC.

- VOICE MESSAGE
- SMS CHARACTER MESSAGE
- E-MAIL

FIG. 11

FIG. 12

START

SET VERTICAL RIGHT POSITION OF HEIGHT MEASURING MODULE — 1101

SENSE WHEN 90% OR MORE OF ULTRASONIC WAVE SIGNALS ARE TRANSMITTED FROM TRANSMITTING UNIT TO RECEIVING UNIT — 1102

STANDBY FOR MEASURING HEIGHT — 1103

1104
CONTACT WITH HAND AND FOOT ELECTRODES

1105
NORMAL CONTACT?  NO

YES  1106
TRANSMIT NORMAL CONTACT STATE SIGNAL TO HEIGHT MEASURING MODULE

FINISH STANDBY — 1107

END

# FIG. 13

START

MEASURE ONE LEAD ECG — 1201

LEAD DROP OR MEASUREMENT ERROR? — 1202

YES

NO

ECG TEMPLATE MATCHING? — 1203

NO

YES

RETRIEVE CORRESPONDING USER DATA — 1300

END

# FIG. 14

```
                    ( START )
                        │
        ┌───────────────┤
        │               ▼
       NO      ╱─────────────────╲
   ◄───────   ╱  NORMAL CONTACT   ╲ ── 1401
             ╲  TO HAND AND FOOT  ╱
              ╲   ELECTRODES?    ╱
               ╲───────────────╱
                        │ YES
    ┌───────────────────┤
    │           ┌────────────────┐
    │           │ MEASURE HEIGHT │ ── 1402
    │           └────────────────┘
    │                   │
    │           ┌────────────────┐
    │           │ MEASURE WEIGHT │ ── 1403
    │           └────────────────┘
    │                   │
    │           ┌────────────────┐
    │           │ MEASURE BODY FAT│ ── 1404
    │           └────────────────┘
    │                   │
   YES     ╱─────────────────╲
   ◄──────╱ MEASUREMENT ERROR? ╲ ── 1405
           ╲───────────────────╱
                    │ NO
            ┌────────────────┐
            │  TRANSMIT AND  │ ── 1600
            │OUTPUT MEASURED DATA│
            └────────────────┘
                    │
                ( END )
```

# FIG. 15

## FIG. 16

## FIG. 17